# EUROPEAN PATENT SPECIFICATION

(11) **EP 3 160 427 B1**
(45) Date of publication and mention of the grant of the patent: **13.09.2017**
(21) Application number: 15727370.7
(22) Date of filing: 01.06.2015
(51) Int. Cl.: A61K 8/27, A61K 8/49, A61Q 7/00, A61K 8/86

(54) **HAIR TREATMENT COMPOSITION**
ZUSAMMENSETZUNG ZUR HAARBEHANDLUNG
COMPOSITIONS DE TRAITEMENT CAPILLAIRE

(30) Priority: 24.06.2014 WO PCT/CN2014/080666; 29.08.2014 EP 14182856
(43) Date of publication of application: 03.05.2017
(73) Proprietor: UNILEVER N.V., 3013 AL Rotterdam (NL); Unilever PLC, London, Greater London EC4Y 0DY (GB)
(72) Inventor: CHEN, Xiaojing, Shanghai 200335 (CN); JAYASWAL, Amit, Bihar 811201 (IN); SHEN, Ying, Shanghai 200335 (CN)
(74) Representative: Chisem, Janet
(86) International application number: PCT/EP2015/062170
(87) International publication number: WO 2015/197317

(56) References cited:
- EP-A1- 2 540 291
- US-A- 4 814 351
- US-A1- 2010 119 461
- DATABASE GNPD [Online] MINTEL; March 2005 (2005-03), Intercosma: "Trattamento Rinforzante Hair Treatment", XP002736043, Database accession no. 347551
- DATABASE GNPD [Online] MINTEL; February 2012 (2012-02), Cosmetiques: "Anti-Hairloss Shampoo", XP002736044, Database accession no. 1739073

## Description

### Field of the invention

The present invention relates to hair treatment compositions. In particular the present invention relates to hair treatment compositions comprising zinc salt, non-ionic surfactant and antimicrobial urea derivate.

### Background of the invention

Loss of hair (hair fall) is a major concern for adults around the world. Thus there has been intensive scientific investigation into the causes of hair fall over many years.

The final step in the hair shedding (exogen) phase of hair fall has been shown to involve a specific proteolitic step, with expression of significant chymotryptic- and tryptic-like proteolytic activities (Milner et al., J Invest Dermatol., 2002, 119, pp.639-644). Thus anti-hair fall products which contain trypsin inhibitors have been developed.

International patent application published as WO 2010/121922 A1 discloses compositions useful for treating or preventing hair fall which comprise an azole fungicide and zinc gluconate. The combination of zinc gluconate and azole is shown to have an anti-trypsin effect.

While compositions such as those described in WO 2010/121922 A1 represent a significant development in anti-hair fall treatments, the present inventors have recognized a need for improved compositions. In particular the present inventors have recognized a need for compositions that not only are effective at combating hair fall but which also have improved properties such as, for example, in-use sensory properties and/or storage stability.

### Summary of the invention

In a first aspect, the present invention is directed to a hair treatment composition comprising:
a) from 0.00001 to 1% zinc salt by weight of the composition;
b) from 0.2 to 2% non-ionic surfactant by weight of the composition; and
c) a preservative system comprising an antimicrobial urea derivative,
wherein the preservative system additionally comprises iodopropynyl butylcarbamate in an amount of from 0.0001 to 0.01% by weight of the composition. In a further aspect, the present invention provides use of the composition of any embodiment of the first aspect for preventing or reducing hair fall. This aspect may alternatively be described in any of the following ways:
- A method of preventing or reducing hair fall in an individual in need thereof, comprising topically applying the composition of any embodiment of the first aspect to the hair and/or scalp of the individual.
- The composition of any embodiment of the first aspect for use as a medicament, preferably for use as a medicament in the treatment or prevention of hair fall.
- Use of the composition of any embodiment of the first aspect in the manufacture of a medicament for the treatment or prevention of hair fall.

In a still further aspect, the invention provides a method of directing an individual to apply the composition of any embodiment of the first aspect to the hair and/or scalp of the individual in order to prevent or reduce hair fall.

All other aspects of the present invention will more readily become apparent upon considering the detailed description and examples which follow.

### Detailed description

The hair treatment composition of the present invention comprises zinc salt. The present inventors have found that a range of salts containing zinc ions are capable of inhibiting trypsin activity and therefore have potential utility in combating hair fall.

There is no particular limitation as to the zinc salt suitable for use in the present invention, save that it is suitable for application to human hair and/or scalp. The zinc salt may, for example, be selected from one or more of zinc acetate, zinc ammonium sulfate, zinc ascorbate, zinc bromide, zinc carbonate, zinc chloride, zinc citrate, zinc fluoride, zinc formate, zinc glycerophosphate, zinc iodide, zinc lactate, zinc nitrate, zinc oxalate, zinc phosphate, zinc salicylate, zinc slufate, zinc succinate, zinc sulphite, and zinc tartrate. References herein to salts should be taken to mean the salt in any form including, for example, hydrate form.

The present inventors have found that more soluble zinc salts have better efficacy at inhibiting trypsin. Thus it is preferred that the salt is selected from slightly soluble zinc salt, soluble zinc salt and mixtures thereof, most preferably the zinc salt is soluble. As used herein the term "soluble" means a salt that has a solubility in water at 20 °C and 1 atm of greater than 1 g salt / 100 ml water. "Insoluble" means a salt that has a solubility in water at 20 °C and 1 atm of less than 0.01 g salt / 100 ml water. Therefore "slightly soluble" means a salt that has a solubility in water at 20 °C and 1 atm of from 0.01 to 1 g / 100 ml.

The especially preferred zinc salt for use in the present invention comprises one or more of zinc chloride, zinc nitrate, zinc sulfate, and zinc gluconate, most preferably comprises or is zinc sulfate.

The amount of zinc salt included in the composition is from 0.00001 to 1% by weight of the composition. The non-ionic surfactant and preservative system is capable of stabilizing the composition even at relatively high levels of zinc salt, thus the composition may comprise at least 0.00005%, more preferably at least 0.0001, more preferably still at least 0.001% and most preferably at least 0.01% zinc salt by weight of the composition. To avoid instability of the composition it is additionally or alternatively preferred that the amount of zinc salt is not too high. Preferably the composition may comprise less than 0.5%, more preferably less than 0.2%, and most preferably less than 0.1 % zinc salt by weight of the composition.

The present inventors have found that the stability of zinc-containing hair treatment compositions can be optimized by inclusion of the non-ionic surfactant and the preservative system comprising an antimicrobial urea derivative.

The urea derivative for use in the preservative system is preferably diazolidinyl urea, imidazolidinyl urea or a mixture thereof, most preferably diazolidinyl urea. The preservative system preferably comprises the urea derivative in an amount of from 0.05 to 0.5% by weight of the composition. The preservative system additionally comprises iodopropynyl butylcarbamate, in an amount of from 0.0001 to 0.01% by weight of the composition.

The composition comprises the non-ionic surfactant in an amount of from 0.2 to 2% by weight of the composition, preferably from 0.3 to 1.7% and most preferably 0.4 to 1.5%. The non-ionic surfactant preferably comprises one or more of polyoxyethylene glycol alkyl ether, polyoxypropylene glycol alkyl ether, glucoside alkyl ether, polyoxyethylene glycol octylphenol ether, polyoxyethylene glycol alkylphenol ether, glycerol alkyl esters, polyoxyethylene glycol sorbitan alkyl esters, sorbitan alkyl esters, cocamide MEA, cocamide DEA, dodecyldimethylamine oxide, block copolymer of polyethylene glycol and polypropylene glycol, polyethoxylated tallow amine.

The most preferred non-ionic surfactant is polyoxyethylene glycol alkyl ether, especially the polyoxyethylene glycol alkyl ether with the INCI designation Oleth 20. Oleth 20 is available, for example, from Croda International plc under the trade name Brij™ 020.

Regardless of the total amount of non-ionic surfactant, it is preferred that the composition comprises at least 0.2%, more preferably at least 0.3% and most preferably at least 0.4% by weight of the composition of one or more of polyoxyethylene glycol alkyl ether, polyoxypropylene glycol alkyl ether, glucoside alkyl ether, polyoxyethylene glycol octylphenol ether, polyoxyethylene glycol alkylphenol ether, glycerol alkyl esters, polyoxyethylene glycol sorbitan alkyl esters, sorbitan alkyl esters, cocamide MEA, cocamide DEA, dodecyldimethylamine oxide, block copolymer of polyethylene glycol and polypropylene glycol, polyethoxylated tallow amine. In one embodiment the composition comprises at least 0.2%, more preferably at least 0.3% and most preferably from 0.4% to 1% by weight of the composition of polyoxyethylene glycol alkyl ether, especially Oleth 20.

The present inventors have found that the non-ionic surfactant and preservative system of the invention can stabilize zinc-containing compositions, even where the composition contains perfume oils. Thus in one embodiment the hair treatment composition comprises perfume, more preferably the composition comprises perfume in an amount of from 0.01 to 1% by weight of the composition, even more preferably from 0.03 to 0.5% and most preferably from 0.05 to 0.2%.

Opacifiers may also be included in the composition as in some circumstances they may also contribute to stability and/or sensory properties. Exemplary opacifiers include, for example, latex of styrene and/or styrene copolymer. Most preferred is the latex with the INCI name Styrene/Acrylates Copolymer and sold, for example, by the Dow Chemical Company under the trade name Opulyn™ 301. The composition preferably comprises opacifier in an amount of from 0.001 to 5% by weight of the composition, more preferably from 0.01 to 2%, more preferably still from 0.05 to 1% and most preferably from 0.1 to 0.6%.

In one embodiment the hair treatment composition of the present invention comprises polyacrylate. As used herein the term "polyacrylate" refers to those non-crosslinked acrylate polymers with the INCI designation Polyacrylate rather than cross-linked acrylate-type polymers which have the INCI name Carbomer or whose INCI name includes the designation "Crosspolymer". Typically the polyacrylate is a homopolymer of 2-Propenoic acid and/or its salt. Suitable salts include, for example, sodium polyacrylate, ammonium polyacrylate, potassium polyacrylate or a mixture thereof.

The present inventors have found that hair treatment compositions comprising polyacrylate have superior sensory performance than those containing acrylate crosspolymer. In particular consumer tests have shown that a leave-on formulation comprising polyacrylate was rated superior by consumers to a leave on treatment comprising acrylate crosspolymer in the following sensory attributes:
- It does not leave my hair or scalp sticky (female consumers);
- It does not leave scalp greasy (female consumers);
- It does not leave residue on scalp (male consumers).

Such sensory parameters are especially important for compositions intended to combat hair fall as not only is it important that a user applies the product in the correct manner, but also that the user is willing to apply the product with the necessary frequency to achieve anti-hair fall benefits.

In order to maximize the sensory benefits provided by the polyacrylate it is preferred that the hair treatment composition comprises at least 0.001% polyacrylate by weight of the composition, more preferably at least 0.01%, more preferably still at least 0.05% and most preferably at least 0.1%. Additionally or alternatively it is preferred that the composition comprises no more than 2% polyacrylate by weight of the composition, more preferably no more than 1%, more preferably still no more than 0.5%, and most preferably no more than 0.3%.

The present inventors also found that in order to maintain the desirable sensory properties and/or required product stability, it is desirable when polyacrylate is present to compound the polyacrylate and zinc salt in a specific ratio. Thus the weight ratio of polyacrylate to zinc (II) ions in the composition is preferably from 10:1 to 20000:1. More preferably the weight ratio of polyacrylate to zinc(II) ions is less than 5000:1, still more preferably less than 2000:1, and most preferably less than 1000:1. Additionally or alternatively it is preferred that the weight ratio of polyacrylate to zinc (II) ions is greater than 15:1, more preferably greater than 20:1, more preferably still greater than 40:1, even more preferably greater than 80:1, and most preferably greater than 180:1.

In order to provide even better in-use and/or post-use sensory properties it is preferred that the composition additionally comprises one or more conditioning agents. Exemplary conditioning agents include, for example, cationic conditioning polymers, cationic surfactants, silicone oils, fatty alcohols, liquid hydrocarbons and mixtures thereof.

Cationic conditioning polymers include, for example, cationic galactomannans and polymers generally having the designation Polyquaternium. Especially preferred for use in the present invention is Polyquaternium 11 (which is a copolymer of vinylpyrrolidone and quaternized dimethylaminoethyl methacrylate). Amounts of cationic conditioning polymer preferably range from 0.01 to 2% by weight of the composition, more preferably the composition comprises the cationic conditioning polymer in an amount of from 0.05 to 1.5% by weight of the composition, most preferably from 0.1 to 1%.

An especially preferred liquid hydrocarbon is decene polymer, especially hydrogenated polydecene. Preferably the composition comprises the hydrocarbon in an amount of from 0.001 to 5% by weight of the composition, more preferably from 0.01 to 2% and most preferably from 0.05 to 1%.

The balance of the hair treatment composition will typically be made up by solvent. Exemplary solvents include lower alcohols (i.e. methanol, ethanol, propanol, isopropanol and mixtures thereof), water, volatile silicones and combinations thereof. Surprisingly, however, the present inventors have found that excellent products can be formulated without the use of volatile solvents. Thus in one embodiment the composition is substantially free from volatile solvents, especially lower alcohols. More preferably the composition comprises less than 10% lower alcohol by weight of the composition, more preferably less than 5%, even more preferably less than 2% and most preferably from 0 to 0.5%.

The most preferred solvent for use in compositions of the present invention is water. Preferably the hair treatment composition comprises water in an amount of at least 85% by weight of the composition, more preferably at least 87%, more preferably still at least 90%, even more preferably at least 92% and most preferably from 95 to 99 wt%.

The pH of the composition (at 25 °C) is preferably greater than pH 5, more preferably greater than pH 5.5 and most preferably in the range 6.0 to 8.0.

The viscosity of the composition is not particularly limited. It is, however, preferred that the composition is not too viscous as this may induce an unwanted "sticky" feel during application. Preferably therefore the composition has a viscosity less than 1000 mPa· s, more preferably less than 500 mPa· s, more preferably still less than 200 mPa· s, even more preferably less than 100 mPa· s, and most preferably in the range of 5 to 60 mPas. Viscosities referred to herein are determined at 30 °C using a Brookfield D220 viscometer employing a T2 spindle No. 2 at a speed of 30 rpm for 60 s.

In order to allow maximum opportunity for the zinc salt to contact the hair and/or scalp and deliver a anti-hair fall benefit, it is preferred that the composition is a leave-on composition, i.e., a composition intended to be applied to the hair and/or scalp without a rinsing step. The leave-on composition is preferably left on the hair for at least one hour, more preferably from 2 to 24 hours before rinsing and/or washing the hair.

Typically leave-on compositions are substantially free from cleansing surfactants. Thus it is preferred that the composition comprises less than 1 wt% cleansing surfactant, more preferably less than 0.5 wt%, more preferably still less than 0.1% and most preferably from 0 to 0.01 wt%. Cleansing surfactants are typically anionic surfactants such as, for example, alkyl sulfates and/or alkyl ether sulfates.

Except in the examples, or where otherwise explicitly indicated, all numbers in this description indicating amounts of material or conditions of reaction, physical properties of materials and/or use may optionally be understood as modified by the word "about".

All amounts are by weight of the final hair treatment composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Examples

### Example 1

This example demonstrates the anti-trypsin activity of a range of zinc salts.

### Materials

Trypsin was supplied by SIGMA chemical co.
Zinc Gluconate had a purity of greater than 97% and was supplied by Purac Biochem. Zinc Chloride was analytical grade supplied by Sinopharm Chemical Reagent co.
Zinc Sulfate Heptahydrate had a purity of greater than 99.7% and was supplied by Shi Si He Wei Chemical Industry co.

### Method

Typsin activity was assessed using a commercial assay employing fluorescence measurement (EnzChek^{®} Protease Assay Kit E6639 from Molecular Probes). The assayed solutions contained trypsin (100 µg/ml) and various amounts of the zinc salts in 50 mM Tris-HCl (pH 8.0).

### Results

The trypsin inhibition efficacy as a function of salt concentration is shown in Table 1.

**TABLE 1**

| **Zinc Salt** | **Salt concentration (wt%)** | **Zinc (II) concentration (mg kg⁻¹)** | **Protease inhibition (%)** |
|---|---|---|---|
| Zinc Gluconate | 0.0001 | 0.14 | -4 |
| | 0.001 | 1.4 | 10 |
| | 0.01 | 14 | 28 |
| | 0.1 | 140 | 94 |
| | 1 | 1400 | 94 |
| Zinc Chloride | 0.0001 | 0.48 | 7 |
| | 0.001 | 4.8 | 20 |
| | 0.01 | 48 | 69 |
| | 0.1 | 480 | 93 |
| | 1 | 4800 | 96 |
| Zinc Sulfate Heptahydrate | 0.0001 | 0.23 | 4 |
| | 0.001 | 2.3 | 14 |
| | 0.01 | 23 | 33 |
| | 0.1 | 230 | 92 |
| | 1 | 2300 | 93 |

### Example 2

This example demonstrates the effect of amount of non-ionic surfactant and preservative system on the storage stability of leave-on hair care compositions containing zinc salts.

Samples were prepared using the base formulation indicated in Table 2 but with varying amounts of Oleth 20, perfume, opacifier and with various preservative systems.

**TABLE 2**

| Ingredient | %w/w |
|---|---|
| Deionized water | To 100 |
| Polyquaternium 11 | 0.70 |
| PEG/PPG-25/25 Dimethicone | 1.0 |
| Sodium Polyacrylate | 0.19 |
| Hydrogenated Polydecene | 0.12 |
| Trideceth-6 | 0.02 |
| Oleth-20 | Table 3 |
| Styrene/Acrylates Copolymer | Table 3 |
| Preservative System | Table 3 |
| Perfume | Table 3 |
| Zinc Gluconate | Table 3 |
| Zinc Sulfate Heptahydrate | Table 3 |

The samples were stored at various temperatures between -4 °C and 45 °C for twelve weeks and at 50 °C for 1 week. Samples were also subjected to 10 cycles of freeze-thaw testing. Samples were inspected after storage for signs of defects such as precipitation, bulk phase separation and/or formation of a surface oil layer. The results are shown in Table 3 (amounts are in wt% of the composition). Samples were only considered stable if they passed all of the tests (i.e. showed no defects after 12 week storage, 1 week 50 °C storage and freeze-thaw).

**TABLE 3**

| Sample | ZS¹ | ZG² | Perfume | SAC³ | O 20⁴ | Preservative System | | | | | | Stable? |
|---|---|---|---|---|---|---|---|---|---|---|---|---|
| | | | | | | DU⁵ | IPBC⁶ | PE⁷ | CG⁸ | EDTA⁹ | BA¹⁰ | |
| 1 | 0.018 | 0.0001 | 0.10 | 0.38 | 0.5 | 0.2 | 0.002 | --- | --- | --- | --- | Y |
| A | --- | 0.0020 | 0.10 | 0.38 | 0.1 | 0.2 | 0.002 | --- | --- | --- | --- | N |
| B | --- | 0.0020 | --- | 0.38 | 0.1 | --- | 0.010 | 0.7 | --- | 0.05 | --- | N |
| C | --- | 0.0020 | --- | 0.38 | 0.1 | --- | --- | 0.7 | 0.4 | 0.05 | --- | N |
| D | --- | 0.0010 | 0.15 | 0.38 | 0.1 | --- | --- | 0.7 | 0.4 | 0.05 | --- | N |
| E | --- | 0.0010 | 0.15 | --- | 0.1 | --- | --- | 0.7 | 0.4 | 0.05 | --- | N |
| F | --- | 0.0100 | 0.15 | --- | 0.1 | --- | --- | 0.7 | 0.4 | 0.10 | --- | N |
| G | --- | 0.0001 | 0.15 | --- | 0.5 | --- | --- | 0.7 | 0.4 | 0.05 | --- | N |
| H | --- | 0.0001 | 0.15 | --- | 0.1 | --- | --- | 0.4 | 0.4 | 0.05 | --- | N |
| I | 0.018 | 0.0001 | 0.10 | --- | 0.3 | --- | --- | 0.4 | 0.4 | 0.05 | --- | N |
| J | 0.018 | 0.0001 | 0.10 | --- | 0.5 | --- | --- | 0.4 | 0.4 | 0.05 | --- | N |
| K | 0.018 | 0.0001 | 0.10 | 0.38 | 0.5 | --- | --- | 0.4 | 0.4 | 0.05 | 0.5 | N |
| L | 0.018 | 0.0001 | 0.10 | 0.38 | 0.5 | --- | --- | 0.1 | 0.3 | --- | 0.4 | N |
| M | 0.018 | 0.0001 | 0.10 | 0.38 | 0.5 | --- | --- | 0.2 | 0.3 | --- | 0.4 | N |
| N | 0.018 | 0.0001 | 0.10 | 0.38 | 0.5 | --- | --- | 0.2 | 0.3 | --- | 0.3 | N |
| O | 0.018 | 0.0001 | 0.10 | 0.38 | 0.5 | --- | --- | 0.1 | 0.3 | --- | 0.3 | N |

| | | | | | | | | | | | | |
|---|---|---|---|---|---|---|---|---|---|---|---|---|
| 1. Zinc Sulfate Heptahydrate. 2. Zinc Gluconate. 3. Styrene/Acrylates Copolymer. 4. Oleth 20. 5. Diazolidinyl urea. 6. lodopropynyl butylcarbamate. 7. Penoxyethanol. 8. Capryryl glycol. 9. Ethylenediaminetetraacetic acid. 10. Benzyl alcohol. | | | | | | | | | | | | |

The data in Table 3 demonstrate that only the sample containing a preservative system with urea derivative and high levels of non-ionic surfactant (Sample A) was stable.

## Claims

1. A hair treatment composition comprising:
a) from 0.00001 to 1% zinc salt by weight of the composition;
b) from 0.2 to 2% non-ionic surfactant by weight of the composition; and
c) a preservative system comprising an antimicrobial urea derivative;
wherein the preservative system additionally comprises iodopropynyl butylcarbamate in an amount of from 0.0001 to 0.01% by weight of the composition.

2. The hair treatment composition as claimed In claim 1 wherein the urea derivative is diazolidinyl urea, imidazolidinyl urea or a mixture thereof, preferably diazolidinyl urea.

3. The hair treatment composition as claimed in claim 1 or claim 2 wherein the preservative system comprises the urea derivative in an amount of from 0.05 to 0.5% by weight of the composition.

4. The hair treatment composition as claimed in any one of the preceding claims wherein the non-ionic surfactant comprises one or more of polyoxyethylene glycol alkyl ether, polyoxypropylene glycol alkyl ether, glucoside alkyl ether, polyoxyethylene glycol octylphenol ether, polyoxyethylene glycol alkylphenol ether, glycerol alkyl esters, polyoxyethylene glycol sorbitan alkyl esters, sorbitan alkyl esters, cocamide MEA, cocamide DEA, dodecyldimethylamine oxide, block copolymer of polyethylene glycol and polypropylene glycol, polyethoxylated tallow amine.

5. The hair treatment composition as claimed in claim 4 wherein the non-ionic surfactant comprises polyoxyethylene glycol alkyl ether.

6. The hair treatment composition as claimed in claim 5 wherein the polyoxyethylene glycol alkyl ether is Oleth-20.

7. The hair treatment composition as claimed in any one of claims 4 to 6 wherein the composition comprises at least 0.2% by weight of the composition of one or more of polyoxyethylene glycol alkyl ether, polyoxypropylene glycol alkyl ether, glucoside alkyl ether, polyoxyethylene glycol octylphenol ether, polyoxyethylene glycol alkylphenol ether, glycerol alkyl esters, polyoxyethylene glycol sorbitan alkyl esters, sorbitan alkyl esters, cocamide MEA, cocamide DEA, dodecyldimethylamine oxide, block copolymer of polyethylene glycol and polypropylene glycol, polyethoxylated tallow amine.

8. The hair treatment composition as claimed in any one of the preceding claims wherein the composition comprises opacifier.

9. The hair treatment composition as claimed in claim 8 wherein the opacifier comprises latex of styrene and/or styrene copolymer, preferably Styrene/Acrylates Copolymer.

10. The hair treatment composition as claimed in any one of the preceding claims which is a leave-on composition and is substantially free from anionic cleansing surfactant.

11. The hair treatment composition as claimed in claim 10 wherein the composition comprises less than 1% anionic cleansing surfactant by weight of the composition, preferably the composition comprises from 0 to 0.1% anionic cleansing surfactant by weight of the composition.

12. The hair treatment composition as claimed in any one of the preceding claims wherein the composition comprises water in an amount of at least 85 wt%.

13. The hair treatment composition as claimed in claim 12 wherein the composition comprises water in an amount of from 90 to 99 wt%.

14. The composition as claimed in any one of the preceding claims for use in preventing or reducing hair fall.

## Patentansprüche

1. Zusammensetzung zur Haarbehandlung, umfassend:
a) von 0,00001 bis 1 Gewichts-% der Zusammensetzung Zinksalz,
b) von 0,2 bis 2 Gewichts-% der Zusammensetzung nicht-ionisches Tensid und
c) ein konservierendes System, umfassend ein antimikrobielles Harnstoffderivat,
wobei das konservierende System zusätzlich Iodpropinylbutylcarbamat in einer Menge von 0,0001 bis 0,01 Gewichts-% der Zusammensetzung umfasst.

2. Zusammensetzung zur Haarbehandlung, wie im Anspruch 1 beansprucht, wobei das Harnstoffderivat Diazolidinylharnstoff, Imidazolidinylharnstoff oder eine Mischung davon, vorzugsweise Diazolidinylharnstoff, darstellt.

3. Zusammensetzung zur Haarbehandlung, wie im Anspruch 1 oder Anspruch 2 beansprucht, wobei das konservierende System das Harnstoffderivat in einer Menge von 0,05 bis 0,5 Gewichts-% der Zusammensetzung umfasst.

4. Zusammensetzung zur Haarbehandlung, wie in irgendeinem der vorhergehenden Ansprüche beansprucht, wobei das nicht-ionische Tensid ein(en) oder mehrere von Polyoxyethylenglycolalkylether, Polyoxypropylenglycolalkylether, Glucosidalkylether, Polyoxyethylenglycoloctylphenolether, Polyoxyethylenglycolalkylphenolether, Glycerolalkylester, Polyoxyethylenglycolsorbitanalkylester, Sorbitanalkylester, Cocamid MEA, Cocamid DEA, Dodecyldimethylaminoxid, Blockcopolymer vom Polyethylenglycol und Polypropylenglycol, polyethoxyliertes Talgamin umfasst.

5. Zusammensetzung zur Haarbehandlung, wie im Anspruch 4 beansprucht, wobei das nicht-ionische Tensid Polyoxyethylenglycolalkylether umfasst.

6. Zusammensetzung zur Haarbehandlung, wie im Anspruch 5 beansprucht, wobei der Polyoxyethylenglycolalkylether Oleth-20 darstellt.

7. Zusammensetzung zur Haarbehandlung, wie in irgendeinem der Ansprüche 4 bis 6 beansprucht, wobei die Zusammensetzung mindestens 0,2 Gewichts-% der Zusammensetzung ein(en) oder mehrere von Polyoxyethylenglycolalkylether, Polyoxypropylenglycolalkylether, Glucosidalkylether, Polyoxyethylenglycoloctylphenolether, Polyoxyethylenglycolalkylphenolether, Glycerolalkylester, Polyoxyethylenglycolsorbitanalkylester, Sorbitanalkylester, Cocamid MEA, Cocamid DEA, Dodecyldimethylaminoxid, Blockcopolymer von Polyethylenglycol und Polypropylenglycol, polyethoxyliertes Talgamin umfasst.

8. Zusammensetzung zur Haarbehandlung, wie in irgendeinem der vorhergehenden Ansprüche beansprucht, wobei die Zusammensetzung Trübungsmittel umfasst.

9. Zusammensetzung zur Haarbehandlung, wie im Anspruch 8 beansprucht, wobei das Trübungsmittel Latex von Styrol und/oder Styrolcopolymer, vorzugsweise Styrol/Acrylate-Copolymer umfasst.

10. Zusammensetzung zur Haarbehandlung, wie in irgendeinem der vorhergehenden Ansprüche beansprucht, welche eine Leave-on-Zusammensetzung darstellt und im Wesentlichen frei von anionischem Reinigungstensid ist.

11. Zusammensetzung zur Haarbehandlung, wie im Anspruch 10 beansprucht, wobei die Zusammensetzung weniger als 1 Gewichts-% der Zusammensetzung anionisches Reinigungstensid umfasst, wobei die Zusammensetzung vorzugsweise 0 bis 0,1 Gewichts-% der Zusammensetzung anionisches Reinigungstensid umfasst.

12. Zusammensetzung zur Haarbehandlung, wie in irgendeinem der vorhergehenden Ansprüche beansprucht, wobei die Zusammensetzung Wasser in einer Menge von mindestens 85 Gew.-% umfasst.

13. Zusammensetzung zur Haarbehandlung, wie im Anspruch 12 beansprucht, wobei die Zusammensetzung Wasser in einer Menge von 90 bis 99 Gew.-% umfasst.

14. Zusammensetzung, wie in irgendeinem der vorhergehenden Ansprüche beansprucht, zur Verwendung beim Verhindern oder Verringern von Haarausfall.

## Revendications

1. Composition de traitement des cheveux comprenant :
a) de 0,00001 à 1 % de sel de zinc en masse de la composition ;
b) de 0,2 à 2 % de tensioactif non-ionique en masse de la composition ; et
c) un système conservateur comprenant un dérivé d'urée antimicrobien ;
dans laquelle le système conservateur comprend de plus du butylcarbamate d'iodopropynyle dans une quantité de 0,0001 à 0,01 % en masse de la composition.

2. Composition de traitement des cheveux selon la revendication 1, dans laquelle le dérivé d'urée est la diazolidinylurée, l'imidazolidinylurée ou un mélange de celles-ci, de préférence la diazolidinylurée.

3. Composition de traitement des cheveux selon la revendication 1 ou la revendication 2, dans laquelle le système conservateur comprend le dérivé d'urée dans une quantité de 0,05 à 0,5 % en masse de la composition.

4. Composition de traitement des cheveux selon l'une quelconque des revendications précédentes, dans laquelle le tensioactif non-ionique comprend un ou plusieurs d'alkyléther de polyoxyéthylène glycol, alkyléther de polyoxypropylène glycol, alkyléther de glucoside, octylphénoléther de polyoxyéthylène glycol, alkylphénoléther de polyoxyéthylène glycol, esters alkyliques de glycérol, esters alkyliques de polyoxyéthylène glycol sorbitane, esters alkyliques de sorbitane, cocamide MEA, cocamide DEA, oxyde de dodécyldiméthylamine, copolymère séquencé de polyéthylène glycol et polypropylène glycol, amine de suif polyéthoxylée.

5. Composition de traitement des cheveux selon la revendication 4, dans laquelle le tensioactif non-ionique comprend un alkyléther de polyoxyéthylène glycol.

6. Composition de traitement des cheveux selon la revendication 5, dans laquelle l'alkyléther de polyoxyéthylène glycol est Oleth-20.

7. Composition de traitement des cheveux selon l'une quelconque des revendications 4 à 6, dans laquelle la composition comprend au moins 0,2 % en masse de la composition d'un ou plusieurs d'un alkyléther de polyoxyéthylène glycol, alkyléther de polyoxypropylène glycol, alkyléther de glucoside, octylphényléther de polyoxyéthylène glycol, alkylphénoléther de polyoxyéthylène glycol, esters alkyliques de glycérol, esters alkyliques de polyoxyéthylène glycol sorbitane, esters alkyliques de sorbitane, cocamide MEA, cocamide DEA, oxyde de dodécyldiméthylamine, copolymère séquencé de polyéthylène glycol et polypropylène glycol, amine de suif polyéthoxylée.

8. Composition de traitement des cheveux selon l'une quelconque des revendications précédentes, dans laquelle la composition comprend un opacifiant.

9. Composition de traitement des cheveux selon la revendication 8, dans laquelle l'opacifiant comprend un latex de styrène et/ou copolymère de styrène, de préférence un copolymère de styrène/ acrylates.

10. Composition de traitement des cheveux selon l'une quelconque des revendications précédentes qui est une composition sans rinçage et est pratiquement exempte de tensioactif nettoyant anionique.

11. Composition de traitement des cheveux selon la revendication 10, dans laquelle la composition comprend moins de 1 % de tensioactif nettoyant anionique en masse de la composition, la composition comprend de préférence de 0 à 0,1 % de tensioactif nettoyant anionique en masse de la composition.

12. Composition de traitement des cheveux selon l'une quelconque des revendications précédentes, dans laquelle la composition comprend de l'eau dans une quantité d'au moins 85 % en masse.

13. Composition de traitement des cheveux selon la revendication 12, dans laquelle la composition comprend de l'eau dans une quantité de 90 à 99 % en masse.

14. Composition selon l'une quelconque des revendications précédentes pour une utilisation dans la prévention ou la réduction de la chute des cheveux.
